(19) Europäisches Patentamt
European Patent Office
Office européen des brevets

(11) Publication number: **0 290 273**
**A1**

# (12) EUROPEAN PATENT APPLICATION

(21) Application number: **88304131.1**

(51) Int. Cl.[4]: **A 61 B 5/00**

(22) Date of filing: **06.05.88**

(30) Priority: **08.05.87 JP 110462/87**

(43) Date of publication of application:
**09.11.88 Bulletin 88/45**

(84) Designated Contracting States: **DE GB**

(71) Applicant: **HAMAMATSU PHOTONICS K.K.**
**1126-1 Ichino-cho Hamamatsu-shi**
**Shizuoka-ken (JP)**

(72) Inventor: **Suzuki, Susumu**
**Hamamatsu Photonics K.K. No.1126-1 Ichino-cho**
**Hamamatsu-shi Shizuoka (JP)**

**Yahi, Sumio**
**Hamamatsu Photonics K.K. No.1126-1 Ichino-cho**
**Hamamatsu-shi Shizuoka (JP)**

**Hakamata, Naotoshi**
**Hamamatsu Photonics K.K. No.1126-1 Ichino-cho**
**Hamamatsu-shi Shizuoka (JP)**

**Ozaki, Takeo**
**Hamamatsu Photonics K.K. No. 1126-1 Ichino-cho**
**Hamamatsu-shi Shizuoka (JP)**

(74) Representative: **Rackham, Stephen Neil et al**
**GILL JENNINGS & EVERY 53-64 Chancery Lane**
**London WC2A 1HN (GB)**

## (54) Examination apparatus for measuring oxygenation.

(57) An examination apparatus (1) measures the oxygen saturation in blood using near infrared light transmission spectrophotometry by using the fact that the attenuation of near infrared light varies with a heartbeat. The examination apparatus comprises light sources (LD1-LD4) for emitting near infrared radiation at a number of different wavelengths, a light source controller (55), an illumination-side fixture (51), a detection side fixture (52), a transmitted light detector (54), and a computer (2) which controls the apparatus (1) and analyses the results. To measure the oxygen saturation of parts of a body such as organs or the head, the body's heartbeat period is divided into plural cycles, the transmission quantities of radiation transmitted through the head or organ are accumulated at every wavelength and for every cycle, and the variations of transmission quantities with heartbeat are calculated at every wavelength. The oxygen saturation is determined from the above-calculated variations of transmission quantities.

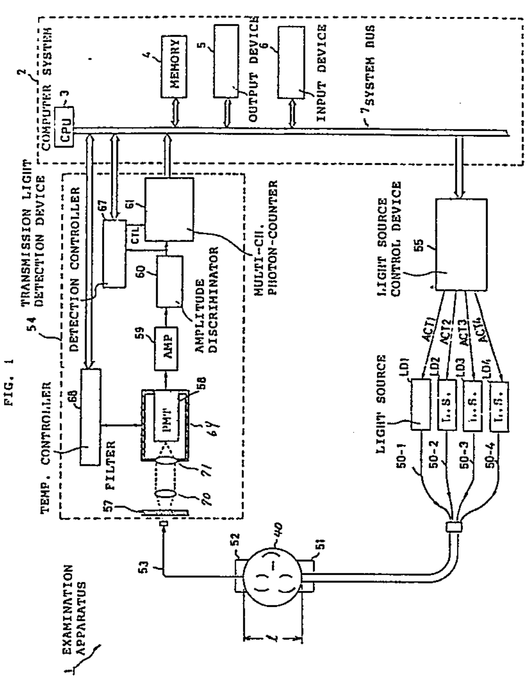


FIG. 1

EP 0 290 273 A1

## Description

### Examination Apparatus For Measuring Oxygenation

The present invention relates to the apparatus for measuring the oxygen concentration in objects such as organs, e.g. the cerebral tissues, of a human body or animals, especially relates to the apparatus for measuring the oxygenation of hemoglobin in blood and that of cytochrome in cells by detecting their effect on electromagnetic radiation.

In general, in diagnosing the function of the body organ, e.g. the cerebral tissues, it is a fundamental and important parameter whether the oxygen quantity in the body organ is sufficient and it is suitably used. Supplying body organs with sufficient quantity of oxygen is indispensable for the growth ability of foetuses and new-born infants. If the supply of oxygen is insufficient, the death rates of foetuses and new-born infants are high, and even if they live serious problems in body organs may remain as a consequence. The insufficiency of oxygen affects every body organ, especially causes a serious damage in the cerebral tissues.

To examine the oxygen quantity in body organs readily and at the early stage of illness, an examination apparatus disclosed in US-A-4,281,645 has been developed. In this kind of examination apparatus, the variation of oxygen quantity in body organs, especially in the brain is measured through the absorption spectrum of near infrared light in which the absorption is caused by the hemoglobin which is an oxygen-carrying medium in blood and the cytochrome a, $a_3$ which performs oxydation-reduction reaction in cells. As shown in Fig. 4(a), the absorption spectra of near infrared light (700 to 1300 nm), $\alpha_{HbO2}$ and $\alpha_{Hb}$, respectively by oxygenated hemoglobin ($HbO_2$) and disoxygenated hemoglobin (Hb) are different from each other. And as shown in Fig. 4(b), the absorption spectra of that, $\alpha_{CyO2}$ and $\alpha_{Cy}$, respectively by oxidized cytochrome a, $a_3$ ($CyO_2$) and reduced cytochrome a, $a_3$ (Cy) are different from each other. This examination apparatus utilizes the above-described absorption spectra of near infrared light. Four near infrared light rays with different wavelengths, $\lambda_1$, $\lambda_2$, $\lambda_3$ and $\lambda_4$ (e.g., 775 nm, 800 nm, 825 nm and 850 nm) are applied to one side of the patient's head with a time-sharing method and the transmission light rays from the opposite side of the head are in turn detected. By processing these four detected results with the prescribed calculation program, four unknown quantities, i.e., the density variations of oxygenated hemoglobin ($HbO_2$), disoxygenated hemoglobin (Hb), oxidized cytochrome a, $a_3$ ($CyO_2$) and reduced cytochrome a, $a_3$ (Cy) are calculated and being based on these parameters, for example the variation of a cerebral oxygen quantity is obtained.

Fig. 5 shows a system outline of the above-described conventional examination apparatus 45. The conventional examination apparatus 45 includes; light sources such as laser diodes LD1 to LD4 which emit four near infrared light rays with different wavelengths of $\lambda_1$, $\lambda_2$, $\lambda_3$ and $\lambda_4$, respectively; a light source control device 55 which controls output timing of the light sources LD1 to LD4; optical fibers 50-1 to 50-4 which introduces near infrared light rays emitted by the light sources LD1 to LD4 to a patient's head 40; an illumination-side fixture 51 which bundles and holds end portions of the optical fibers 50-1 to 50-4; a detection-side fixture 52 which is fitted to the prescribed position of the opposite side of the patient's head 40; a optical fiber 53 which is held by the detection-side fixture 52 and introduces transmitted near infrared light from the patient's head 40; a transmission light detection device 54 which measures transmission quantity of near infrared light by counting photons of near infrared light introduced by the optical fiber 53; and a computer system 56 which controls the total examination apparatus and determines the variation of oxygen quantity in cerebral tissues being based on the transmission quantity of near infrared light.

The computer system 56 is equipped with a processor 62, a memory 63, output devices 64 such as a display and a printer, and an input device 65 such as a keyboard, and these devices are connected each other by a system bus 66. The light source control device 55 and the transmission light detection device 54 are connected to the system bus 66 as external I/O's.

The light source control device 55 drives the light sources LD1 to LD4 by respective driving signals ACT1 to ACT4 as shown in Fig. 6(a) to 6(d) according to instructions from the computer system 56. As shown in Fig. 6 one measuring period $M_k$ ($k = 1, 2, .....$) consists of N cycles of CYl to CYn. At a phase $\Phi n1$ in an arbitrary cycle CYn, no light source of LD1 to LD4 is driven and therefore the patient's head 40 is not illuminated by the near infrared light from the light sources LD1 to LD4. At the phase $\Phi n2$ the light source LD1 is driven and the near infrared light with the wavelength of for example 775 nm is emitted from it. In the same manner, at the phase $\Phi n3$ the light source LD2 is driven and the near infrared light with the wavelength of for example 800 nm is emitted from it; at the phase $\Phi n4$ the light source LD3 is driven and the near infrared light with the wavelength of for example 825 nm is emitted from it; and at the phase $\Phi n5$ the light source LD4 is driven and the near infrared light with the wavelength of for example 850 nm is emitted from it. In this manner the light source control device 55 drives the light sources LD1 to LD4 sequentially with a time-sharing method.

The transmission light detection device 54 is equipped with; a filter 57 which adjusts the quantity of near infrared light outputted from the optical fiber 53; lenses 70 and 71; a photomultiplier tube 58 which converts the light from the filter 57 into pulse current and outputs it; an amplifier 59 which amplifies the pulse current from the photomultiplier tube 58; an amplitude discriminator 60 which eliminates the pulse current from the amplifier 59 whose amplitude is smaller than the prescribed threshold value; a multi-channel photon-counter 61 which detects photon frequency in every channel; for example a detection controller 67 which controls detection periods of the multi-channel photon-counter 61; a temperature controller 68 which controls the temperature of a cooler 69 containing the photomultiplier tube 58.

In use of the above-described examination apparatus, the illumination-side fixture and the detection-side fixture are firmly fitted to the prescribed positions of the patient's head 40 by using a tape or the like. After that, the light sources LD1 to LD4 are driven by the light source control device 55 as shown in Fig. 6(a) to 6(d), respectively, so that the four near infrared light rays with different wavelengths are emitted from the light sources LD1 to LD4 sequentially with the time-sharing method, and the light rays are introduced by the optical fibers 50-1 to 50-4 to the patient's head 40. As bones and soft tissues in the patient's head 40 are transparent to the near infrared light, the near infrared light is partially absorbed mainly by hemoglobin in blood and cytochrome a, $a_3$ in cells and outputted to the optical fiber 53. And the optical fiber 53 introduces the light to the transmission light detection device 54. At the phase Φn1 no light source of LD1 to LD4 is driven, so that the transmission light detection device 54 does not receive the transmission light originally emitted from the light sources LD1 to LD4. At this phase the transmission light detection device 54 detects dark light.

The photomultiplier tube 58 in the transmission light detection device 54 is the one for photon-counting which has high sensitivity and operates at high response speed. The output pulse current from the photomultiplier tube 58 is sent to the amplitude discriminator 60 through the amplifier 59. The amplitude discriminator 60 eliminates the noise component whose amplitude is smaller than the prescribed amplitude threshold and sends only the signal pulse to the multi-channel photon-counter 61. The multi-channel photon-counter 61 detects photon number only in the periods $T_o$ which is made synchronized with driving signals ACT1 to ACT4 for the respective light sources LD1 to LD4 as shown in Fig. 6(a) to (d) by a control signal CTL as shown in Fig. 6(e) from the detection controller 67, and counts detected photon number of every light with each wavelength sent from the optical fiber 53. The transmission data of every near infrared light with each wavelength are obtained through the above-described procedure.

That is, as shown in Fig. 6(a) to (e), at the phase Φn1 in the cycle CYn of light source control device 55 no light source of LD1 to LD4 is driven, therefore the dark light data d are counted by the transmission light detection device 54. At the phases Φn2 to Φn5 the light sources LD1 to LD4 are sequentially driven with the time-sharing method and the transmission light detection device 54 sequentially counts the transmission data $t_{\lambda1}$, $t_{\lambda2}$, $t_{\lambda3}$ and $t_{\lambda4}$ of the respective near infrared light rays with different wavelengths $\lambda_1$, $\lambda_2$, $\lambda_3$ and $\lambda_4$.

The counting of the dark light data d and the transmission data $t_{\lambda1}$, $t_{\lambda2}$, $t_{\lambda3}$ and $t_{\lambda4}$ which is sequentially performed in the cycle CYn, is continued N times from CYl to CYn. That is, one measuring period $M_k$ (k = 1, 2, .....) includes N cycles. A concrete example is as follows; if one cycle is 200 μsec and N is 10000, the measuring period $M_k$ becomes 2 sec. At the time of finishing of one measuring period $M_k$, the counting result of the dark light data D

$$\left( = \sum_{n=1}^{N} d/CYn \right)$$

and the counting results of the transmission data $T_{\lambda1}$, $T_{\lambda2}$, $T_{\lambda3}$ and $T_{\lambda4}$

$$\left( = \sum_{n=1}^{N} t_{\lambda j} \; / \; CYn \right)$$

are transferred to the computer system 56 and stored in the memory 63.

The processor 62 performs the subtraction of the dark light component by using the combination of the transmission data and dark data $(T_{\lambda1}, T_{\lambda2}, T_{\lambda3}, T_{\lambda4}, D)_M$ being stored in the memory 63 after one measuring period $M_k$ and the combination of those $(T_{\lambda1}, T_{\lambda2}, T_{\lambda3}, T_{\lambda4}, D)_{M_o}$ at the start of measuring, and calculations the variation rates of the transmission light, $\Delta T_{\lambda1}$, $\Delta T_{\lambda2}$, $\Delta T_{\lambda3}$ and $\Delta T_{\lambda4}$. That is, the variation rates of the transmission light $\Delta T_{\lambda1}$, $\Delta T_{\lambda2}$, $\Delta T_{\lambda3}$ and $\Delta T_{\lambda4}$ are calculated as:

$\Delta T_{\lambda j} = \log [(T_{\lambda j} - D)_{Mk}/(T_{\lambda j} - D)_{Mo}]$ (j = 1 to 4). (1)

The use of logarithm in the above calculation of $\Delta T_{\lambda j}$ is to express the variation as an optical density.

Using the above-calculated variation rates of the transmission light $\Delta T_{\lambda1}$, $\Delta T_{\lambda2}$, $\Delta T_{\lambda3}$ and $\Delta T_{\lambda4}$, density variations of oxygenated hemoglobin ($HbO_2$), disoxygenated hemoglobin (Hb), oxygenized cytochrome a, $a_3$ ($CyO_2$) and reduced cytochrome a, $a_3$ which are expressed as $\Delta X_{HbO_2}$ $\Delta X_{Hb}$, $\Delta X_{CyO_2}$ and $\Delta X_{Cy}$, respectively, can be determined. That is, each of density variations of $\Delta X_{HbO_2}$ $\Delta X_{Hb}$, $\Delta X_{CyO_2}$ and $\Delta X_{Cy}$ is calculated as:

$$\Delta X_i = \sum_{j=1}^{4} (\alpha_{ij})^{-1} \Delta T_{\lambda j} / \ell \quad \cdots\cdots\cdots\cdots (2)$$

where $\alpha_{ij}$ is an absorption coefficient of each component i ($HbO_2$, Hb, $CyO_2$, Cy) for each wavelength $\lambda j$ ( $\lambda 1$, $\lambda 2$, $\lambda 3$, $\lambda 4$) and is predetermined from Fig. 4(a) and (b), and $\ell$ is the length of the patient's head 40 along the travelling direction of the near infrared light.

As the above-detected density variation components, $\Delta X_{HbO_2}$, $\Delta X_{Hb}$, $\Delta X_{CyO_2}$ and $\Delta X_{Cy}$, reflect the variation of oxygen quantity in the brain, the variation of oxygen quantity in the brain can be known by outputting these detected results from the output device 64 and the diagnosis is made being based on those results.

As is described above, the conventional examination apparatus can measure the variation of oxygen quantity in the prescribed body organ. From the comparison between Fig. 4(a) and (b), it is understood that the measured absorption spectrum is mainly caused by hemoglobin in blood and the contribution of cytochrome a, $a_3$ to the spectrum is very small. Therefore, the measured variation of oxygen quantity in the prescribed body organ is regarded to be mainly caused by the density variation of hemoglobin (Hb or $HbO_2$) in blood.

By the way, it is sometimes required by an user of the examination apparatus to measure the oxygen saturation S, generally defined as:

$S = X_{HbO2}/(X_{HbO2} + X_{Hb})$ (3)

instead of the variation of oxygen quantity. In the equation (3) the denominator and the numerator represent total hemoglobin density and oxygenated hemoglobin ($HbO_2$) density in blood, respectively.

Such an art that measures the oxygen saturation S is described in the paper by I. Yoshiya et al, which is on page 27 to 32 of "Medical & Biological Engineering & Computing, Vol. 18" published in January 1980. According to this paper, the fingertip is illuminated by the light emitted from a halogen light and the oxygen saturation S in arterial blood is determined by utilizing the fact that the transmission quantity of light from the fingertip is modulated being synchronized with the heartbeat. The oxygen saturation of total blood (arterial and venous blood) is calculated according to the Beer's law as:

$S = A - B(\alpha_{650}/\alpha_{805})$ (4)

where $\alpha_{650}$ and $\alpha_{805}$ are the absorption coefficients of total blood at the wavelengths 650 nm and 805 nm, respectively, and A and B are coefficients relating to the absorption coefficients of disoxygenated hemoglobin (Hb) and oxygenated hemoglobin ($HbO_2$), respectively.

The incident light to the fingertip is attenuated by blood in transmitting it. It is assumed that in this process, while the attenuation of light by the arterial blood varies being synchronized with the heartbeat as shown in Fig. 7, the attenuation of light by the venous blood does not vary. Now, defining the logarithmic ratio Y of the total transmission quantity ($E_{DC + AC}$) to the dc component of transmission quantity $E_{DC}$ as:

$Y = log(E_{DC + AC}/E_{DC})$, (5)

the logarithmic ratio Y is proportional to the absorption coefficient $\alpha$. By measuring logarithmic ratios $Y_{650}$ and $Y_{805}$ at the wavelength 650 nm and 805 nm, respectively, the ratio of these logarithmic ratios are obtained. Because, from the above discussion, the equation of:

$Y_{650}/Y_{805} = \alpha_{650}/\alpha_{805}$ (6)

is obtained, the oxygen saturation S is described by using measured $Y_{650}$ and $Y_{805}$ as:

$S = A - B(Y_{650}/Y_{805})$. (7)

As is described above, according to the procedure described in the above-referred paper the oxygen saturation S in the fingertip is obtained by utilizing the fact that the attenuation quantity or the transmission quantity of light by arterial blood is modulated being synchronized with the heartbeat. However, in the case that the body organ to be measured is the head, as the transmission quantity of light is very small and the variation of the transmission quantity by the heartbeat is little, it has been difficult to adopt the procedure of the above-referred paper in the same manner as is described therein.

According to this invention an examination apparatus for measuring oxygenation in a body with electromagnetic radiation transmission spectrophotometry, comprising:

light source means which emits electromagnetic radiation at a number of different wavelengths;

an illumination-side fixture;

a detection-side fixture;

a computer system which controls the apparatus and analyses the measurements;

light source control means which drives the light source means according to an instruction from the computer system; and,

transmitted light detection means which detects transmission quantities of electromagnetic radiation transmitted through the body;

is characterised in that the light source control means drives the light source means so that it sequentially emits the electromagnetic radiation cyclically in a semi-measuring period of a heartbeat period of the body;

in that the transmitted light detection means accumulates the transmission quantities at every wavelength in every semi-measuring period to obtain accumulated transmission quantity data; and,

in that the computer system divides the heartbeat period into the semi-measuring periods and into a plurality of cycles for each semi-measuring period, controls the light source control means, and the transmission light detection means so that the two means operate in synchronism with the cycles, the semi-measuring periods and the heartbeat periods, receives the accumulated transmission quantity data from the transmission light detection means, and calculates the oxygen saturation from the received transmission quantity data by determining variations in the accumulated transmission quantity data at each wavelength.

The present invention provides an examination apparatus which can measure the oxygen saturation with high reliability substantially independently of the body organ being measured.

The examination apparatus according to the invention typically comprises:

division means which divides a heartbeat period into plural cycles;

transmission light detection means which detects transmission quantities of electromagnetic waves with different wavelengths sequentially emitted from light sources in each cycle divided by the division means;

accumulation means which accumulates the detected transmission quantities at the every wavelength and in the above every cycle by repeating the above cycles with the repetition of the heartbeat period;

calculation means which calculates the variation of the transmission quantity in the heartbeat period at the every wavelength from the transmission quantities accumulated by the accumulation means; and operation means which determines the oxygen saturated by performing the prescribed operation for the variations of the transmission quantities calculated by the calculation means.

In the examination apparatus of the invention, the heartbeat period is divided into plural cycles, in each divided cycle the body organ, for example the head, is sequentially illuminated by the electromagnetic waves with different wavelengths from light source means, and the transmission quantities of the electromagnetic waves which are transmitted through the head are detected by the transmission light detection means. The transmission quantities detected in any one cycle in one heartbeat period are very small and the variations of the transmission quantity between cycles is very small but by accumulating the detected transmission quantities at the every wavelength and in the every cycle synchronised with the heartbeat accurate results can be obtained.

Thus in examination apparatus of the invention, the oxygen saturation is precisely determined even in the head or other body organs in which the transmitted quantity of light is small.

Particular embodiments of examination apparatus in accordance with this invention will now be described with reference to the accompanying drawings; in which:-

Figure 1 is a block diagram of an embodiment of the invention;

Figure 2(a) is a time-chart of a heartbeat signal and Figure 2(b) shows the semi-measuring periods;

Figure 3 is a graph of the accumulated values of transmission data which are accumulated over one measuring period;

Figures 4(a) and (b) are graphs illustrating the absorption spectrum of hemoglobin and cytochrome, respectively;

Figure 5 is a block diagram of a conventional examination apparatus;

Figures 6(a) to (e) are time-charts of driving signals ACT1 to ACT4 and a control signal CTL, respectively; and,

Figure 7 is a graph to explain the variation of transmission quantity caused by blood.

Figure 1 shows a system constitution of an examination apparatus which is an embodiment of the invention. Figure 2(a) is a time-chart of a heartbeat signal BT and Figure 2(b) shows semi-measuring period $m_\ell'$ ($1 \leqq \ell \leqq P$). In Figure 1 the same reference numerals or characters are given to the parts as those to the corresponding parts in Figure 5 and the explanation for these parts will be omitted.

In an examination apparatus 1, which is an embodiment of the invention, a computer system 2 has a system bus 7 to which a processor 3, a memory 4, an output device 5 and an input device 6 are connected, in the same manner as the computer system 56 of the conventional examination apparatus 45. And the examination apparatus 1 determines the oxygen saturation S by utilizing the fact that the transmission quantity of near infrared light is modulated being synchronized with the heartbeat, in the same manner as the above-referred paper. The examination apparatus 1 is characterized in that a period of a heartbeat signal BT is divided into P-periods, transmission quantities in each period is accumulated in the repetition and thereby the oxygen saturation is precisely determined. The heartbeat signal BT is sent from the external system through the input device 6.

In the computer system 2 each of the periods S1, $S_2$, . . . . in the heartbeat signal BT, are divided into P-intervals, that is, semi-measuring periods $m_1'$ to $m_p'$. In this embodiment "P" is selected to be "16" and the heartbeat period is equally divided into 16 of semi-measuring periods $m_1'$ to $m_{16}'$. For example, if the heartbeat period S1 is 0.6 sec, each of semi-measuring periods $m_1'$ to $m_{16}'$ becomes 37.5 msec. And if one cycle CYn is selected to be 200 µsec, as is described above, each of light sources LD1 to LD4 emits a light 187.5 times in each of semi-measuring intervals $m_1'$ to $m_{16}'$.

One example is as follows. The near infrared light rays emitted from the light sources LD1 to LD4 being sequentially driven in one cycle CYn ( n = 1 to 187 or 188) in one period S1 are attenuated by the patient's head 40 and sent to the transmission light detection device 54. The multi-channel photon-counter 61 counts photon numbers of the transmission light rays at every wavelength of $\lambda_1$ to $\lambda_4$ and in every semi-measuring period of $m_1'$ to $m_p'$. This counting is continued over plural heartbeat periods until the time when the counted numbers of all measuring units at each wavelength and in each semi-measuring period exceeds the prescribed

value. If it is assumed that this condition is satisfied when 10000 times of light emission have been made in every measuring unit, the measurement is performed over heartbeat times of 10000 / 187, that is about 54 times (about 32 sec), and this corresponds to one measuring period $M_k$ (k = 1, 2, .....). When one measuring period $M_k$ is finished, the multi-channel photon-counter 61 transfers the counting results in every counting channel to the computer system 2. The accumulated transmission quantity data at the wavelength $\lambda_i$ and in the semi-measuring period $m_\ell$ is expressed as $T_{\lambda 1}(\ell)_{Acc}$, where i = 1 to 4 and $\ell$ = 1 to P.

By accumulating transmission quantity data in every semi-measuring period of $m_1'$ to $m_p'$ in the above-described procedure, the transmission quantity in each of semi-measuring periods $m_1'$ to $m_p'$ becomes large enough to be analyzed and the variation between semi-measuring periods $m_1'$ to $m_p'$ caused by the heartbeat can be clearly detected.

Paying attention to one wavelength $\lambda_1$, Fig. 3 shows the variation of the accumulation values $T_{\lambda 1}(1)_{Acc}$, $_{\lambda 1}(2)_{Acc}$,....., $T_{\lambda 1}(P)_{Acc}$, which are obtained by accumulating over one measuring period $M_k$ the transmission quantity data $T_{\lambda 1}(1)$, $T_{\lambda 2}(2)$,..... $T_{\lambda 1}(P)$ detected in every semi-measuring period of $m_1'$ to $m_p'$. The accumulation values of transmission quantity data for the other wavelengths $\lambda_2$, $\lambda_3$ and $\lambda_4$ also vary in the same manner as Fig. 3. In Fig. 3, the maximum transmission quantity $F_{DC+AC}$ ($\lambda$1) and the dc component of transmission quantity $F_{DC}(\lambda_1)$ of the accumulation values $T_{\lambda 1}(1)_{Acc}$, $T_{\lambda 2}(1)_{Acc}$,....., $T_{\lambda 1}(P)_{Acc}$, corresponds to the total transmission quantity $E_{DC+AC}$ and the dc component of transmission quantity $E_{DC}$ in the above-referred paper, respectively. Therefore, the logarithmic ratio $Y_{\lambda 1}$ of the maximum transmission quantity $F_{DC+AC}(\lambda_1)$ to the dc component of transmission quantity $F_{DC}(\lambda_1)$ is calculated in the same manner as the equation (5), that is:

$Y_{\lambda 1} = \log(F_{DC} + {}_{AC}(\lambda_1)/F_{DC}(\lambda_1))$. (8)

The logarithmic ratios $Y_{\lambda 2}$, $Y_{\lambda 3}$ and $Y_{\lambda 4}$ for the other respective wavelengths $\lambda_2$, $\lambda_3$ and $\lambda_4$ are calculated in the same manner.

In this embodiment of the invention, the oxygen saturation S, which is generally defined as the equation (3), is calculated being based on the logarithmic ratios $Y_{\lambda 1}$, $Y_{\lambda 2}$, $Y_{\lambda 3}$ and $Y_{\lambda 4}$ calculated from the equation (8). That is, using the logarithmic ratios $Y_{\lambda 1}$, $Y_{\lambda 2}$, $Y_{\lambda 3}$ and $Y_{\lambda 4}$, the equation (3) is expressed as:

$$S = \sum_{i=1}^{4} a_i \cdot Y_{\lambda i} / \sum_{i=1}^{4} b_i \cdot Y_{\lambda i}, \quad \cdots\cdots\cdots\cdot (9)$$

where $a_i$ and $b_i$ are the coefficients which are uniquely determined by the above-described absorption coefficient matrix $\alpha_{ij}$. The oxygen saturation S in blood, in arterial blood in more detailed expression, is calculated by substituting the logarithmic ratios $Y_{\lambda 1}$, $Y_{\lambda 2}$, $Y_{\lambda 3}$ and $Y_{\lambda 4}$ obtained from the equation (8) for those in the equation (9).

Though near infrared light rays with four different wavelengths $\lambda_1$ to $\lambda_4$ are used in the above embodiment, the number of wavelengths is not limited to four, that is, the number may be two or more than four. Moreover, the electromagnetic waves with different wavelengths may be obtained by using one white light source and filtering white light emitted from it. And, the electromagnetic wave emitted from the light source is not limited to near infrared light, that is, far infrared light, visible light, microwave, etc. may be used.

## Claims

1. An examination apparatus (1) for measuring oxygenation in a body with electromagnetic radiation transmission spectrophotometry, comprising:

light source means (LD1-LD4) which emits electromagnetic radiation at a number of different wavelengths;

an illumination-side fixture (51);

a detection-side fixture (52);

a computer system (2) which controls the apparatus and analyses the measurements;

light source control means (55) which drives the light source means (LD1-LD4) according to an instruction from the computer system (2); and,

transmitted light detection means (54) which detects transmission quantities of electromagnetic radiation transmitted through the body;

characterised in that the light source control means (55) drives the light source means so that it sequentially emits the electromagnetic radiation cyclically in a semi-measuring period of a heartbeat period of the body;

in that the transmitted light detection means (54) accumulates the transmission quantities at every wavelength in every semi-measuring period to obtain accumulated transmission quantity data; and,

in that the computer system (2) divides the heartbeat period into the semi-measuring periods and into

a plurality of cycles for each semi-measuring period, controls the light source control means (55), and the transmission light detection means (54) so that the two means (54, 55) operate in synchronism with the cycles, the semi-measuring periods and the heartbeat periods, receives the accumulated transmission quantity data from the transmission light detection means (54), and calculates the oxygen saturation from the received transmission quantity data by determining variations in the accumulated transmission quantity data at each wavelength.

2. An examination apparatus as claimed in claim 1, wherein the electromagnetic radiation is near infrared light radiation.

3. An examination apparatus as claimed in claim 2, wherein the light source means comprises plural laser diodes (LD1-LD4) which emit respectively near infrared light radiation of different wavelengths.

4. An examination apparatus as claimed in any preceding claim, wherein the heartbeat period is obtained by a heartbeat signal sent from an external system which measures the heartbeat of the body.

5. An examination apparatus for measuring the oxygenation in a body organ with electromagnetic wave transmission spectrophotometry, having light source means which emits plural electromagnetic waves with different wavelengths, an illumination-side fixture and a detection-side fixture, comprising:

light source control means which drives said light source means according to an instruction from a computer system so that said light source means sequentially emits said electromagnetic waves in each cycle in a semi-measuring period in a heartbeat period;

transmission light detection means which detects transmission quantities of said electromagnetic waves transmitted from said body organ and accumulates said transmission quantities at said every wavelength and in said every semi-measuring period to obtain accumulated transmission quantity data; and

a computer system which divides said heartbeat period into said plural cycles and into said semi-measuring periods so as for said each semi-measuring period to contain said plural cycles, controls said light source control means through said instruction and said transmission light detection means so that said two means operate being synchronized with said cycles, said semi-measuring periods and said heartbeat periods, receives said accumulated transmission quantity data from said transmission light detetion means and calculates the oxygen saturation from said received transmission quantity data through determining of variations of said accumulated transmission quantity data at each wavelength.

FIG. 1

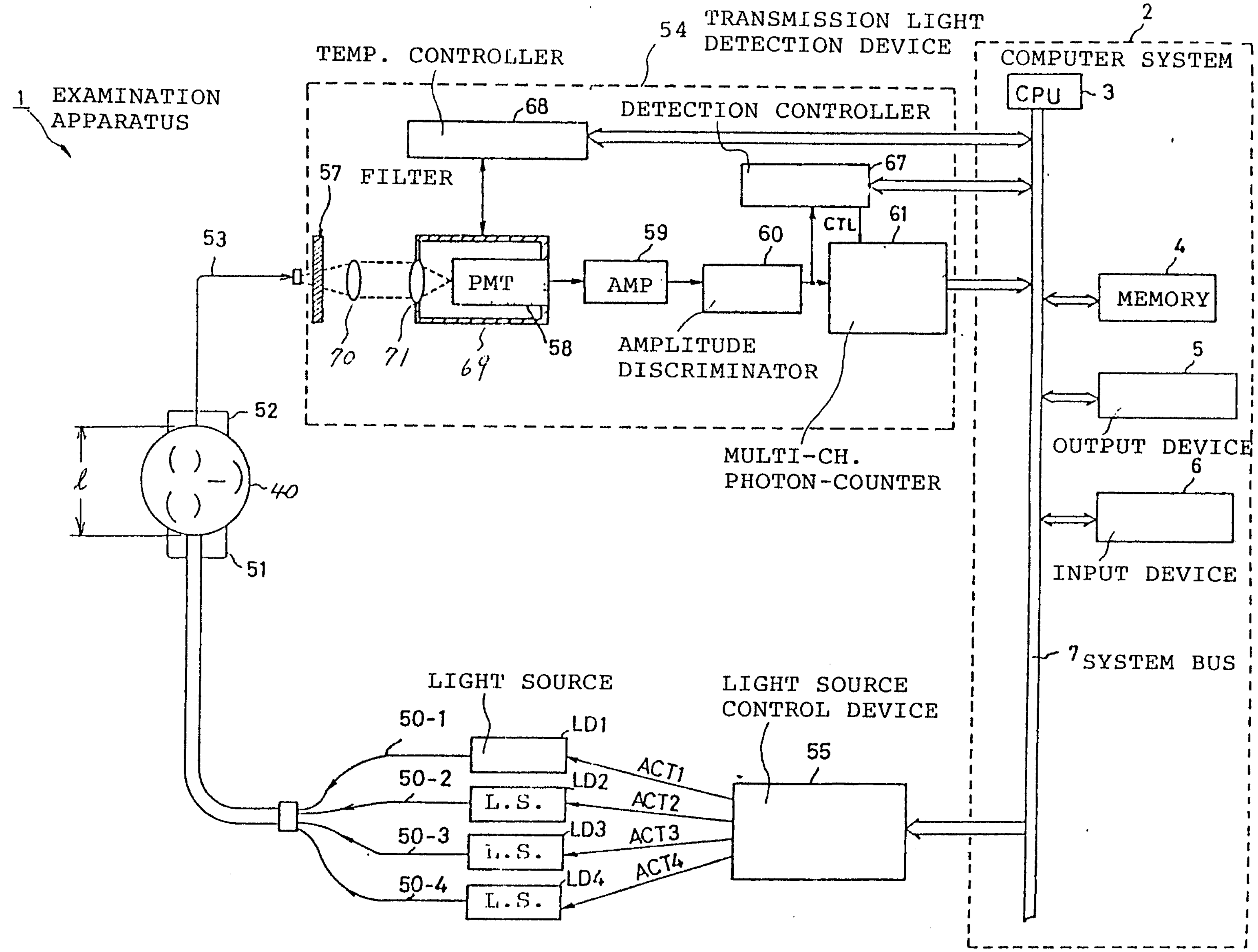

1 EXAMINATION APPARATUS
TEMP. CONTROLLER
54 TRANSMISSION LIGHT DETECTION DEVICE
2 COMPUTER SYSTEM
CPU 3
68
DETECTION CONTROLLER
67
57 FILTER
CTL
61
53
59
60
PMT
AMP
4
MEMORY
70
71
69
58
AMPLITUDE DISCRIMINATOR
5
52
OUTPUT DEVICE
6
ℓ
40
MULTI-CH. PHOTON-COUNTER
51
INPUT DEVICE
7 SYSTEM BUS
LIGHT SOURCE
LIGHT SOURCE CONTROL DEVICE
50-1
LD1
55
ACT1
50-2
LD2
L.S.
ACT2
50-3
LD3
L.S.
ACT3
50-4
LD4
L.S.
ACT4

0290273

FIG. 2

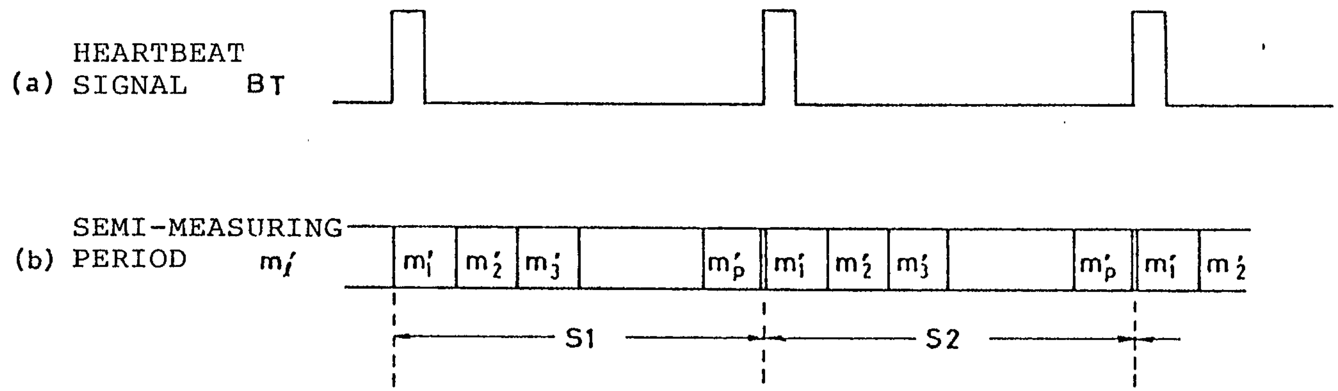


FIG. 3

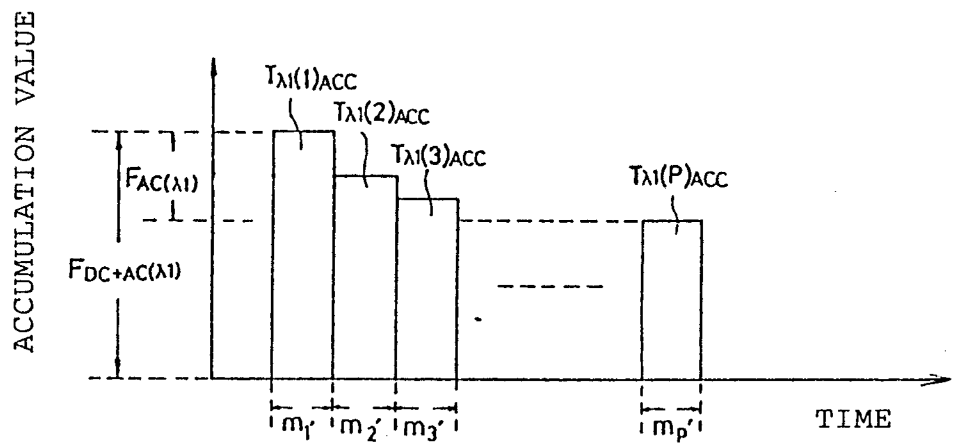

FIG. 4

(a)

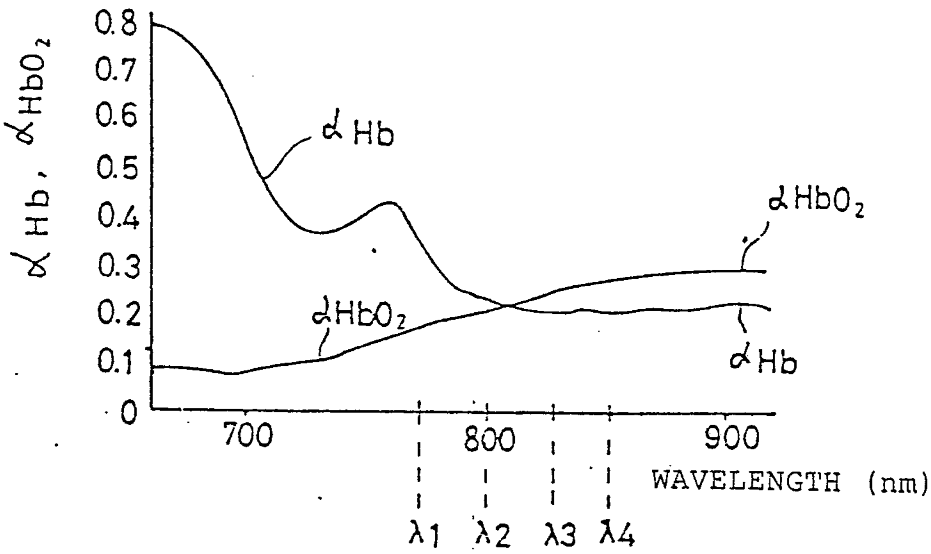
αHb, αHbO2
0.8
0.7
0.6
0.5
0.4
0.3
0.2
0.1
0
αHb
αHbO2
αHbO2
αHb
700
800
900
WAVELENGTH (nm)
λ1
λ2
λ3
λ4

(b)

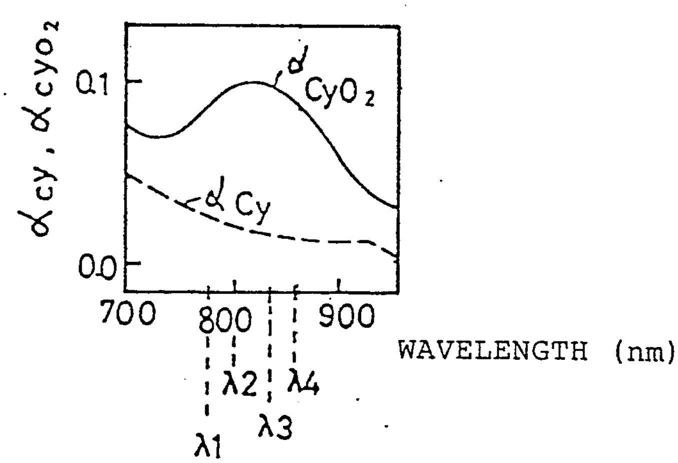
αcy, αcyo2
0.1
0.0
αCyO2
αCy
700
800
900
WAVELENGTH (nm)
λ1
λ2
λ3
λ4

0290273

FIG. 5

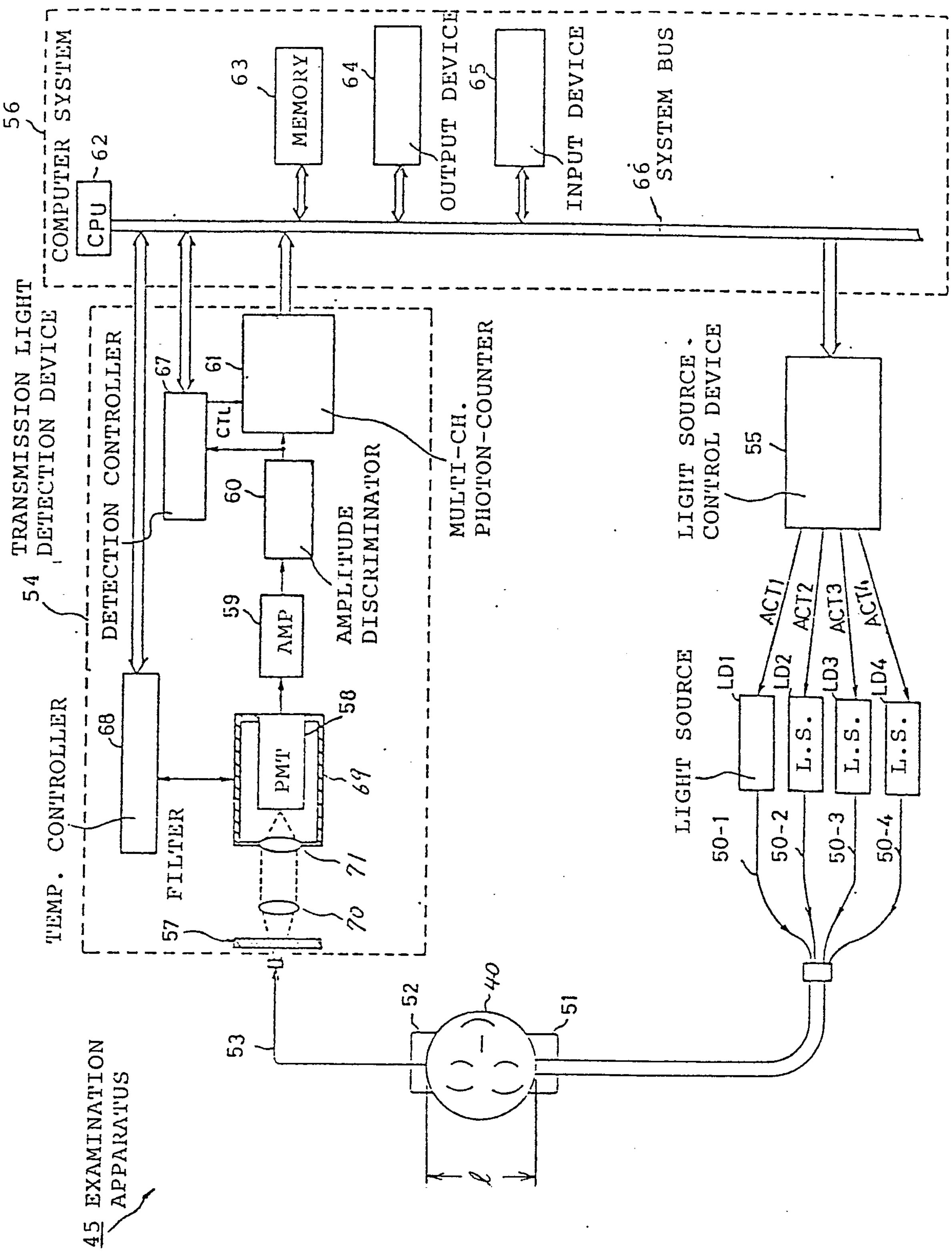

FIG. 6

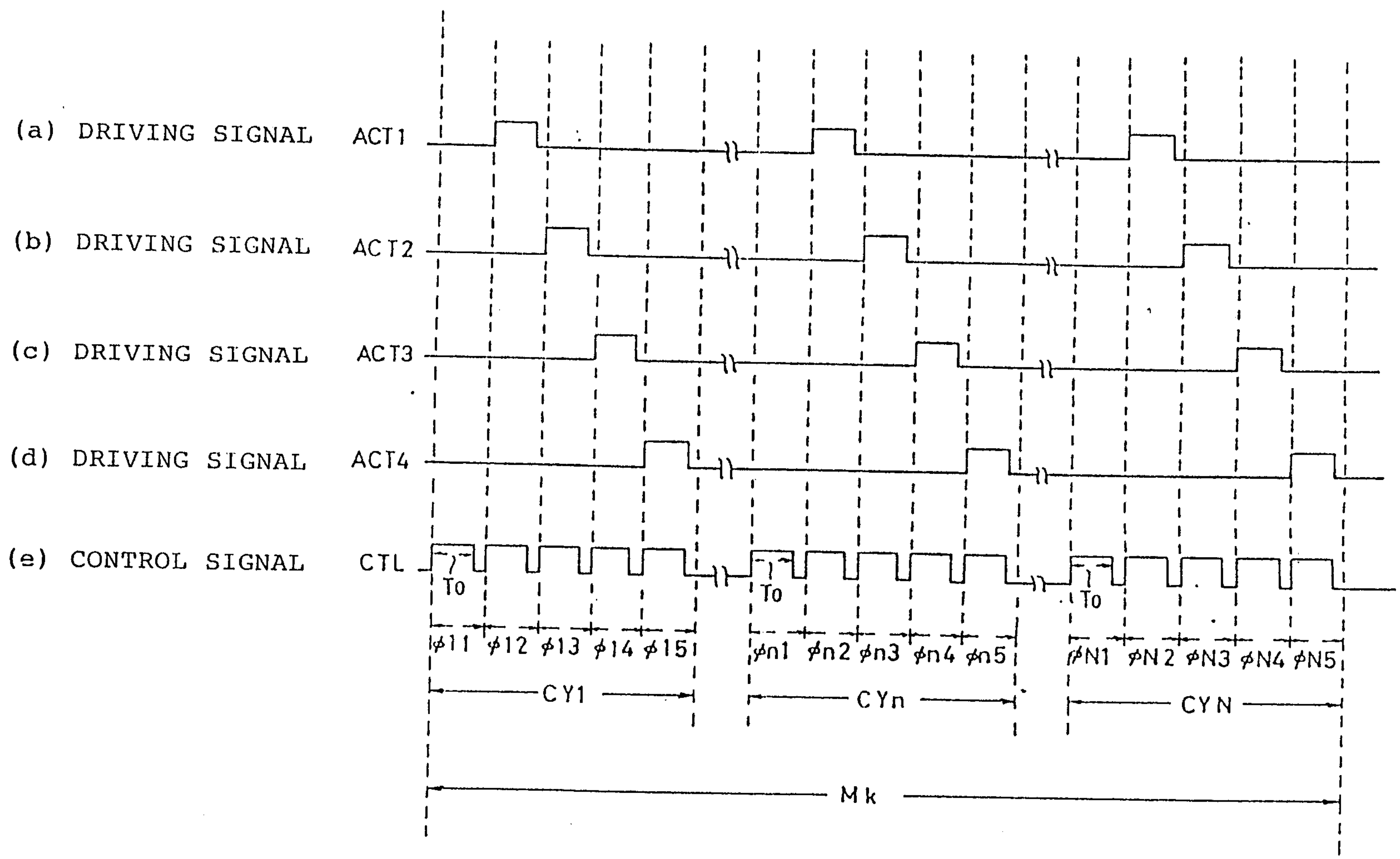
(a) DRIVING SIGNAL ACT1
(b) DRIVING SIGNAL ACT2
(c) DRIVING SIGNAL ACT3
(d) DRIVING SIGNAL ACT4
(e) CONTROL SIGNAL CTL
To
φ11 φ12 φ13 φ14 φ15
φn1 φn2 φn3 φn4 φn5
φN1 φN2 φN3 φN4 φN5
CY1
CYn
CYN
Mk

FIG. 7

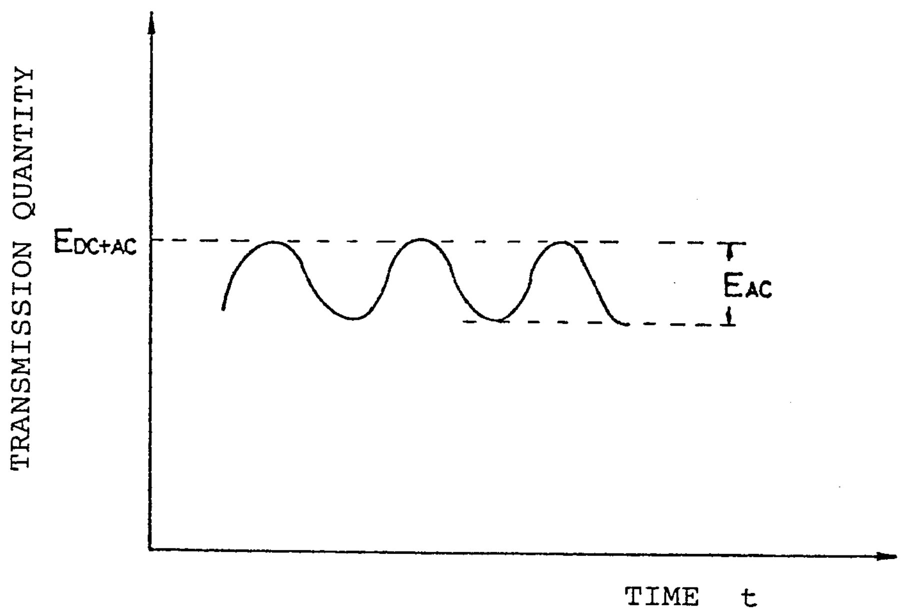

European Patent Office

# EUROPEAN SEARCH REPORT

Application Number

EP 88 30 4131

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y,D | US-A-4 281 645 (F.F. JÖBSIS)<br>* Whole document * | 1,5 | A 61 B 5/00 |
| A | --- | 2-4 | |
| Y | JOURNAL OF NUCLEAR MEDICINE, vol. 16, no. 1, January 1975, pages 95-98, New York, US; M.V. GREEN et al.: "High temporal resolution ecg-gated scintigraphic angiocardiography"<br>* Pages 95,96, paragraph "Method" *<br>--- | 1,5 | |
| A | IEEE TRANS. ON NUCLEAR SCIENCE, vol. 28, no. 1, February 1981, pages 127-130, IEEE, New York, US; S.G. BURGISS et al.: "Physiological and real-time gating of data for positron emission computed tomography"<br>* Whole article *<br>----- | 1,5 | |

TECHNICAL FIELDS SEARCHED (Int. Cl.4)

A 61 B
G 01 N

The present search report has been drawn up for all claims

| Place of search | Date of completion of the search | Examiner |
|---|---|---|
| THE HAGUE | 02-08-1988 | FERRIGNO, A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document